Europäisches Patentamt

European Patent Office

Office européen des brevets

(19)

(11) **EP 1 544 599 A2**

(12) **DEMANDE DE BREVET EUROPEEN**

(43) Date de publication:
**22.06.2005 Bulletin 2005/25**

(51) Int Cl.$^7$: **G01N 15/08**

(21) Numéro de dépôt: **04292754.1**

(22) Date de dépôt: **23.11.2004**

(84) Etats contractants désignés:
**AT BE BG CH CY CZ DE DK EE ES FI FR GB GR
HU IE IS IT LI LU MC NL PL PT RO SE SI SK TR**
Etats d'extension désignés:
**AL HR LT LV MK YU**

(30) Priorité: **19.12.2003 FR 0315199**

(71) Demandeur: **Institut Français du Pétrole
92852 Rueil-Malmaison Cedex (FR)**

(72) Inventeur: **Fleury, Marc
78170 La Celle Saint Cloud (FR)**

(54) **Méthode et dispositif pour mesurer des caractéristiques physiques d'un échantillon solide poreux**

(57)     -Méthode pour mesurer des caractéristiques physiques d'au moins un échantillon solide poreux (S) saturé avec un premier fluide en réalisant des phases de drainage ou d'imbition, en présence d'un deuxième fluide de masse spécifique différente de celle du premier fluide.
-   On place un échantillon saturé avec un premier fluide tel que de la saumure dans un godet rempli d'un second fluide. Au moyen d'un ensemble de centrifugation, on draine l'échantillon jusqu'à établir une saturation finale au sein de l'échantillon. Dans un deuxième temps, on peut procéder à une imbition de l'échantillon. Les phases de drainage ou d'imbition sont réalisées en interposant une plaque poreuse (1) entre l'échantillon (S) et le godet où il est placé, cette plaque poreuse étant mouillable par le premier fluide ou le deuxième fluide avec de préférence une pression d'entrée supérieure à la pression capillaire la plus élevée imposée à la face de l'échantillon en contact avec la plaque poreuse et perforée avec des perforations en nombre et avec une section adaptés pour permettre un drainage rapide du fluide contenu dans la carotte et obtenir un profil de saturation sensiblement uniforme.
-   Applications notamment pour des mesures pétrophysiques.

FIG. 1

## Description

La présente invention concerne une méthode et un dispositif de centrifugation pour mesurer des caractéristiques physiques d'un échantillon solide poreux.

## ETAT DE LA TECHNIQUE

Dans les programmes d'analyse spéciale d'échantillons de roche ou carottes prélevées dans un milieu tel qu'une zone souterraine, la mise en place d'une saturation en eau initiale représentative joue un rôle clé dans la préparation des carottes. Il s'agit de mettre en place les fluides dans des proportions représentatives de celles présentes à l'origine dans la zone réservoir après la migration de l'huile. Typiquement, si l'on souhaite étudier l'efficacité de l'injection d'eau et mesurer la courbe de pression capillaire et de perméabilité relative, la saturation initiale Swi est importante et doit être représentative des conditions in situ. Le terme « initiale » est utilisé ici à dessein pour éviter toute confusion avec le terme «irréductible» qui décrit la saturation asymptotique obtenue avec une pression capillaire élevée pour un ensemble de fluides donné. Dans une zone de transition, ces deux saturations sont très différentes.

Selon une procédure standard, les échantillons sont extraits de carottes plein diamètre, puis nettoyés avec des solvants appropriés. Les échantillons sont ensuite amenés à la saturation initiale (Swi) ou la saturation irréductible (Swirr), selon leur emplacement en termes de pression capillaire et vieillis avec de l'huile brute. A ce stade, la quantité d'eau présente joue également un rôle déterminant pour l'obtention d'un état de mouillabilité représentatif. C'est pourquoi un effort important est en général réalisé pour la mise en place de cette saturation initiale (Swi).

Plusieurs techniques connues permettent de parvenir à cet état. On peut par exemple confiner l'échantillon saturé d'eau dans une cellule et procéder au déplacement de cette eau par une injection d'huile. Il est toutefois connu qu'il est difficile d'obtenir de faibles saturations en eau, essentiellement du fait de la présence d'hétérogénéités, malgré la mise en oeuvre d'une huile visqueuse (typiquement 50 cp). Une huile visqueuse peut également s'avérer peu pratique pour de faibles perméabilités. La saturation moyenne peut de ce fait être encore élevée après la percée et la production résiduelle peut être significative et prendre plusieurs jours. En outre, le profil de saturation est fortement non-uniforme, comme c'est le cas avec la centrifugation standard. Ce profil peut toutefois être réduit en inversant le sens de l'injection. Bien que la centrifugation constitue la technique la plus efficace pour désaturer les carottes, elle n'a pas été mise en oeuvre pour établir la saturation (Swi) à cause de la présence d'un fort profil de saturation qui peut donner lieu à des problèmes d'interprétation lors d'expériences ultérieures d'injection. On peut citer, à titre d'exemple de technique de substitution, la méthode de séchage, pour laquelle la principale difficulté consiste à contrôler ou à imposer la salinité et le profil de saturation. L'idéal serait d'utiliser un processus de déplacement par capillarité, comme c'est le cas in situ, avec un temps expérimental compatible avec le calendrier du programme de développement ou d'évaluation, qui est généralement court. Afin d'éviter les profils non-uniformes et d'obtenir une faible saturation en eau, la méthode de la plaque poreuse peut être mise en oeuvre à la place des techniques mentionnées ci-dessus. Les expériences prennent beaucoup de temps (de l'ordre de quelques semaines à quelques mois), notamment dans le cas de carottes de grande longueur. De plus, le contact capillaire entre la carotte et la plaque poreuse est souvent difficile à optimiser et peut aboutir à un faible taux de réussite.

Enfin, la technique de centrifugation constitue probablement la solution la plus attractive. Il s'agit d'un procédé de déplacement dominé par la capillarité, rapide et peu coûteux, qui présente un certain nombre d'avantages pratiques. Son principal inconvénient réside toutefois dans la non-uniformité du profil de saturation et, pour certaines centrifugeuses, dans la limite imposée en matière de longueur des carottes.

## LA METHODE ET LE DISPOSITIF SELON L'INVENTION

La méthode selon l'invention permet de mesurer des caractéristiques physiques d'un échantillon solide poreux saturé avec un premier fluide en réalisant des phases de drainage ou d'imbibition, en présence d'un deuxième fluide de masse spécifique différente que celle du premier fluide. Elle comporte l'utilisation d'un ensemble de centrifugation comportant au moins un godet pour l'échantillon, adapté à appliquer à l'échantillon une force centrifuge orientée suivant la direction d'allongement de manière à déplacer le premier fluide par le deuxième fluide et la détermination de la valeur finale de la saturation en fluide déplacé.

Les phases de drainage ou d'imbibition sont réalisées en appliquant une plaque poreuse contre une face terminale de l'échantillon traversée par le fluide déplacé, cette plaque poreuse étant mouillable par le fluide déplacé et étant perforée avec des perforations en nombre et avec une section adaptés pour permettre un drainage rapide du fluide contenu dans la carotte et obtenir un profil de saturation sensiblement uniforme.

Si la phase déplacée est la plus lourde (de l'eau par exemple dans une phase de drainage), on interpose une plaque poreuse entre l'échantillon et son support dans le godet. Si la phase déplacée est la plus légère, (comme de l'huile dans une phase d'imbibition), on place cette plaque poreuse contre l'échantillon, du côté le plus proche de l'axe de rotation de l'ensemble de centrifugation.

La plaque poreuse perforée est réalisée de manière à avoir de préférence une pression d'entrée supérieure à la pression capillaire la plus élevée imposée à la face de l'échantillon en contact avec la plaque poreuse.

Suivant un mode de mise en oeuvre, on réalise une phase de drainage d'un échantillon saturé par un premier liquide (L1) en le plaçant en contact avec une plaque poreuse perforée mouillable par le premier fluide, disposée du côté de l'échantillon le plus éloigné de l'axe de rotation de l'ensemble de centrifugation.

La plaque poreuse est réalisée par exemple dans un matériau tel que de la céramique poreuse utilisée habituellement dans les expériences de pression capillaire ou tout autre matériau dont les propriétés sont équivalentes tels qu'un ciment poreux notamment.

Suivant un autre mode de mise en oeuvre, on réalise une phase d'imbibition de l'échantillon drainé en le plaçant en contact avec une plaque poreuse perforée mouillable par le deuxième fluide disposé du côté de l'échantillon le plus proche de l'axe de rotation de l'ensemble de centrifugation.

Dans le cadre de cette mise en oeuvre, on utilise une plaque poreuse perforée réalisée par exemple dans un matériau poreux et perméables tel que le Téflon.

Le dispositif selon l'invention permet de mesurer des caractéristiques physiques d'au moins un échantillon solide poreux saturé avec un premier fluide en réalisant des phases de drainage ou d'imbibition, en présence d'un deuxième fluide de masse spécifique plus faible que celle du premier fluide. Il comporte un ensemble de centrifugation comprenant au moins un godet allongé pourvu d'une chambre pour un échantillon, chaque godet étant fixé à l'extrémité d'un bras solidaire d'un axe de rotation, des moyens moteurs pour entraîner le bras en rotation et créer une force centrifuge orientée suivant la direction d'allongement du godet, et des moyens pour déterminer la valeur finale de la saturation en fluide déplacé.

Le dispositif comporte également une plaque poreuse au contact de l'échantillon, cette plaque étant perforée avec des perforations en nombre et avec une section adaptés pour permettre un drainage rapide du fluide contenu dans la carotte et obtenir un profil de saturation sensiblement uniforme.

De préférence, la plaque poreuse est réalisée de manière à avoir une pression d'entrée supérieure à la pression capillaire la plus élevée avec la face de l'échantillon en contact avec la plaque poreuse.

La méthode et le dispositif présentés ici sont très efficaces pour établir une saturation irréductible à une pression capillaire représentative du champ considéré. La saturation en huile résiduelle (Sor) sera donc également représentative car elle dépend de (Swi) et, ce qui est également important, la mouillabilité sera représentative car elle dépend de la quantité d'eau présente dans le système poreux.

## PRESENTATION DES FIGURES

Les caractéristiques et avantages de la méthode et du dispositif selon l'invention, apparaîtront plus clairement à là lecture de la description ci-après d'un exemple non limitatif de réalisation, en se référant aux dessins annexés où :

- la figure 1 montre un schéma de principe d'un appareillage permettant la mise en oeuvre de la méthode;

- la figure 2A montre des exemples de variation de la pression capillaire Pc en fonction de la saturation en eau Sw;

- les figures 2B, 2C et 2D montrent pour différents paliers de vitesse, des exemples de profil de saturation calculé sans plaque poreuse (A) et avec plaque poreuse (B) (système air-eau), tout le long d'une carotte ;

- la figure 3 montre les lignes d'écoulement du fluide selon qu'elles correspondent à une zone 2 en face d'une perforation ou une zone 1 en face d'une partie non perforée, zones présentant des pressions capillaires différentes ;

- les figures 4A à 4D montrent différents exemples de variations obtenues par balayage CT aux rayons X, de la saturation Sw tout le long d'une carotte en grès avec plaque poreuse (Fig.4A) et sans plaque poreuse (Fig.4B) et d'une carotte en carbonate (Fig.4C, 4D) avec plaque poreuse (Fig.4C) et sans plaque poreuse (Fig.4D), et ceci pour différentes vitesses de rotation, ; le profil de saturation final est uniforme pour le grès ; pour le carbonate compact ;

- la figure 5 montre un pied capillaire (CF) détecté par des mesures de temps de relaxation par RMN ; l'existence d'une bosse pour le temps de relaxation de la carotte saturée à 100 % indique un profil non-uniforme ;

- la figure 6 montre par comparaison deux courbes de variation de Sw en fonction du temps obtenues avec plaque poreuse (A) et sans plaque poreuse (B) pour deux petites carottes (air/eau) ;

- la figure 7 montre la production observée lorsque le niveau huile-eau est déplacé rapidement depuis la face de sortie (Rmax-d, figure 1) d'une carotte de grès (K=610 mD, φ=23 %, L=5,9 cm) jusqu'à la face de sortie de la plaque poreuse (Rmax) soumise à centrifugation ; et

- la figure 8 montre un exemple d'appareil de centrifugation permettant la mise en oeuvre de la méthode.

## DESCRIPTION DETAILLEE DE L'INVENTION

### Principe

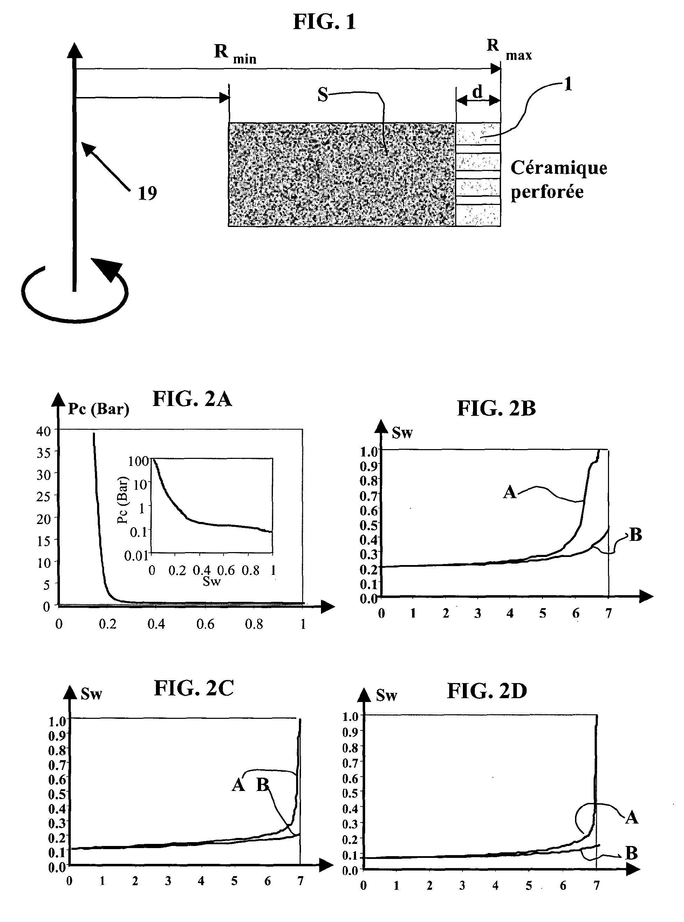

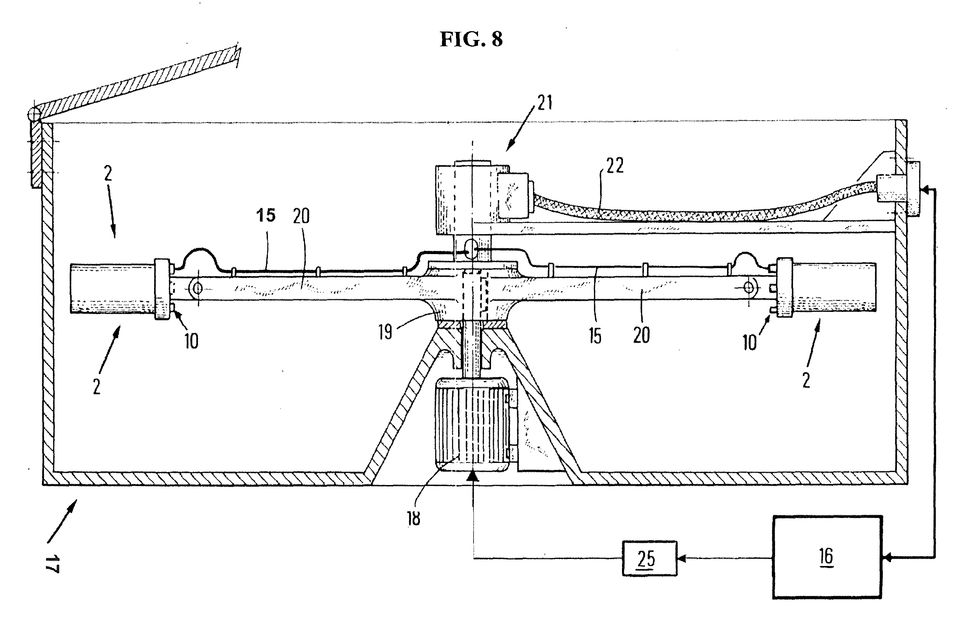

La méthode est mise en oeuvre en utilisant un dispositif de centrifugation tel que décrit par exemple dans les brevets FR 2699 282 (US 5 463 894), FR 2 763 690 ou FR 2 772 477 (US 6 185 985) du demandeur, que l'on a schématisé à la figure 1 et plus en détail à la figure 8.

Un dispositif de centrifugation convenant pour la mise en oeuvre de la méthode comporte par exemple (Fig. 8) une cuve 17, un moteur électrique 18 dont l'axe entraîne en rotation un moyeu 19. Deux (ou quatre) bras 20 identiques sont montés en opposition deux à deux sur le moyeu 19. Des récipients ou godets 2 sont montés pivotants aux extrémités de chacun des bras 20 de façon à s'aligner spontanément avec la direction de la force centrifuge appliquée, et ils s'équilibrent l'un l'autre en rotation. L'échantillon à évaluer, est placé dans au moins un des godets 2. Des moyens (non représentés) placés dans chaque godet, permettent de mesurer les déplacements progressifs de l'interface entre les deux liquides au cours des opérations de drainage et de réimbibition.

Des câbles 15 associés aux différents moyens de mesure, sont connectés à un connecteur électrique tournant multi-lignes 21 d'un type connu porté par le moyeu 19. Le stator de ce tournant 21 est connecté par un câble 22, à un appareil extérieur 16 adapté à commander la variation par paliers de la vitesse de rotation du moteur par l'intermédiaire d'une interface de commande 25, et à traiter les signaux émanant des moyens de mesure dans les godets 2.

L'échantillon S saturé avec un liquide L1 (de la saumure par exemple) est placé dans un godet contenant un autre fluide L2 de densité différente (tel que de l'huile). Par la mise en rotation du bras tournant, on lui applique une force centrifuge de façon à étudier les déplacements des fluides dans l'échantillon au cours d'au moins deux phases distinctes. Durant une première phase de drainage, on soumet alors l'ensemble à une force centrifuge dirigée suivant la longueur du récipient de manière à exercer sur lui une force d'expulsion qui tend à faire sortir une partie du premier fluide L1. Dans le même temps, du fluide L2 pénètre à l'intérieur de l'échantillon. Les deux fluides se déplacent à l'intérieur de l'échantillon jusqu'à une position d'équilibre où la force due à la pression capillaire dans les pores, compense la force centrifuge exercée.

A partir de la mesure précise de la quantité du fluide initial extraite à la fin de la centrifugation, on pourra déterminer la saturation moyenne de l'échantillon et cette saturation sera pratiquement uniforme le long de l'échantillon.

Pour la mise en oeuvre spécifique de la méthode, l'échantillon ou carotte de longueur L est placée dans le godet 2 en appui à sa base (à une distance Rmax-d de l'axe de rotation du bras tournant) sur une plaque poreuse perforée d'épaisseur d (Fig.1 mouillable par le premier fluide L1. Cette plaque poreuse peut être réalisée par exemple en céramique poreuse, en ciment poreux, etc. Sa pression d'entrée est suffisamment élevée pour empêcher que le fluide L2 ou de l'air ne pénètre dedans. Son extrémité opposée est à la distance Rmin de ce même axe. La plaque poreuse permet d'obtenir un profil de saturation quasiment uniforme (élimination du pied capillaire) et les perforations permettent de préserver un débit aussi élevé que possible au cours de la centrifugation.

Compte non tenu des effets 2D, la pression capillaire en un rayon r est donnée par :

$$P_c(r) = \frac{1}{2}\ \omega^2 \Delta\rho (R_{max}^2 - r^2) \tag{1}$$

où $\Delta\rho$ est la différence de masse volumique entre l'eau et l'air ou l'huile, et $\omega$ est la vitesse de rotation de la centrifugeuse. En admettant un contact capillaire entre la carotte et la plaque poreuse, l'équation 1 indique simplement que la pression capillaire au niveau de la face de sortie (Rmax-d) de la carotte est différente de zéro. Ainsi la face de sortie de la carotte sera désaturée en fonction de la courbe de pression capillaire.

A partir de l'équation 1, nous pouvons exprimer le rapport de pression capillaire à l'entrée Pc(Rmin) et à la sortie Pc(Rmax-d) de la carotte :

$$\frac{Pc\ entrée}{Pc\ sortie} = \frac{R_{max}^2 - R_{min}^2}{R_{max}^2 - (R_{max} - d)^2} \tag{2}$$

Typiquement, la plaque poreuse présente une épaisseur de 1 cm et le rapport mentionné ci-dessus s'élèvera à 6,1 et 8,7 respectivement pour L=6 et 10 cm respectivement (Rmax=25 cm). Pour une courbe de pression capillaire donnée, ce rapport exprime indirectement la saturation minimale et maximale dans la carotte.

Pour illustrer l'effet de la plaque poreuse, nous avons calculé les profils de saturation à différentes vitesses de rotation, figures 2B, 2C et 2D, avec (B) et sans (A) cette plaque, pour une pression capillaire mesurée (figure 2A, Sw=f(Pc) et Pc=f(r) d'après l'équation 1). Lorsque la vitesse de rotation est suffisante, le profil de saturation est presque constant car la totalité de la carotte est à une pression correspondant à la partie asymptotique de la courbe Pc.

Sur l'exemple de profil de saturation calculé avec et sans plaque poreuse (système air-eau) montré sur les figures 2B, 2C et 2D, la pression capillaire a été déduite par injection de mercure. Du fait de la plaque poreuse, le profil Sw est presque uniforme à vitesse élevée (>2500 t/min). La face d'entrée est située à un rayon Rmin=17 cm (x=0 le long de la carotte).

Effet des perforations, conditions aux limites

En premier lieu, la plaque poreuse est réalisée de manière à avoir une pression d'entrée supérieure à la pression capillaire la plus élevée au rayon r=Rmax-d. C'est pourquoi elle n'est jamais désaturée. Une désaturation de la plaque poreuse entraînerait une diminution considérable du débit (effet de perméabilité relative) et des incertitudes au niveau du bilan massique pour le calcul de la saturation, et ne faciliterait pas un bon contact capillaire. Dans le cas présent, la pression d'entrée air-eau de la plaque poreuse est d'environ 3,5 bars (0,35 MPa). L'inconvénient réside dans sa faible perméabilité ($K_c$ est par exemple de l'ordre de 0,2 mD). De ce fait, si elle n'était pas perforée, le débit serait dominé par la plaque poreuse comme le laisse prévoir la relation :

$$\frac{L+t}{K_T} = \frac{L}{K_s} + \frac{t}{K_c} \tag{3},$$

Par exemple, pour une carotte de perméabilité $K_s$ = 100 mD, la perméabilité totale $K_T$ s'élèverait à 1,2 mD, ce qui représente une perte considérable.

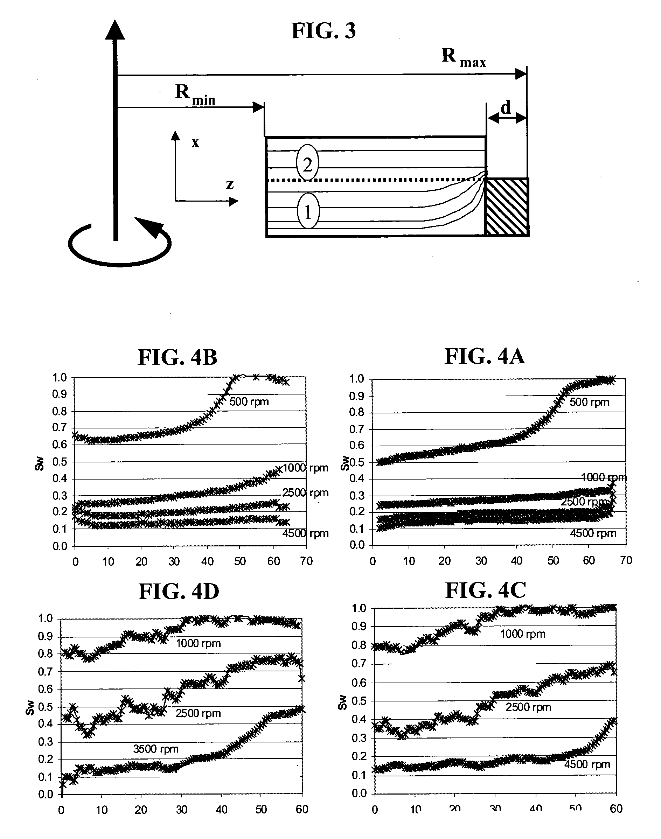

Les perforations permettent à la cinétique du système d'être dominée par la carotte et non par la plaque poreuse comme les tests ont permis de le vérifier. Toutefois, la question des conditions aux limites se pose, et elle n'est pas simple à résoudre. Pour démontrer que les perforations ne modifient pas la pression capillaire au niveau de la face de sortie, nous considérons le système représenté sur la figure 3. Au-dessus d'une perforation (zone 2) et au-dessus de la plaque poreuse (zone 1), les pressions capillaires sont en équilibre vertical selon :

$$P_{C1}(r) = \frac{1}{2} \omega^2 \Delta\rho (R_{max}^2 - r^2) \; et \; P_{C2}(r) = \frac{1}{2} \omega^2 \Delta\rho ((R_{max} - t)^2 - r^2) + C \tag{4}$$

où C est une constante d'intégration inconnue. A un rayon de rotation r donné, une différence de pression capillaire dans les zones 1 et 2 entraînerait un écoulement qui ne peut être équilibré par aucune force dans la direction x. C'est pourquoi, afin d'obtenir l'équilibre dans la direction x, les pressions capillaires Pc1 et Pc2 doivent être égales et de même les saturations dans les zones 1 et 2 doivent être égales. Une désaturation se produit également, notamment au niveau de la face de sortie de la carotte qui ne comporte pas de plaque poreuse (zone 2). Dans la pratique, les perforations ne doivent pas être trop grandes (taille de la zone 2 trop grande par rapport à 1). Pour des raisons liées à des contraintes mécaniques, le diamètre des perforations est de 2 mm et leur nombre a été fixé de manière empirique à 20 (l'écart moyen entre les perforations s'élevant ainsi à environ 0,8 cm). La majeure partie du fluide expulsé hors de la carotte va passer à travers ces perforations. Le choix du nombre de perforations, de leur diamètre, de leur disposition doit satisfaire certaines exigences : maximiser la surface de contact entre l'échantillon et la plaque poreuse pour assurer à celle-ci une bonne tenue mécanique, tout en permettant une bonne évacuation des fluides.

Aspects pratiques

En pratique, la technique SPP présente plusieurs avantages :

- la manipulation est simple et de nombreuses carottes peuvent être désaturées simultanément (6 dans le cas présent) ; grâce à l'utilisation de centrifugeuses de grand diamètre (Rmax=25 cm) et à la mise en oeuvre d'une vitesse intermédiaire ($\omega_{max}$=4900 t/min), il est possible d'utiliser des carottes dont la longueur peut atteindre 12 cm et une pression capillaire élevée peut être obtenue (64 bars air-eau, 31 bars dodécane-eau),

- le contact capillaire est facile à obtenir (si les faces de la carotte sont planes) car les forces centrifuges poussent la carotte contre la plaque poreuse.

La principale difficulté consiste à ne pas endommager les carottes, notamment celles qui présentent une longueur importante. Il peut dans certains cas s'avérer impossible d'obtenir un profil de saturation uniforme lorsque la vitesse de rotation doit être limitée. Il existe deux solutions pour contourner ce problème : (i) utiliser une plaque poreuse plus longue afin de réduire le rapport Pc entrée/Pc sortie (équation 2) et minimiser la vitesse de rotation et/ou (ii) retourner la carotte et faire tourner à la même vitesse pendant une durée similaire.

La méthode peut s'appliquer indifféremment au drainage air/eau ou au drainage huile/eau. Le drainage air/eau présente toutefois certains avantages par rapport au drainage huile/eau. Tout d'abord, comme la différence de masse volumique est plus importante, une vitesse inférieure est requise, ce qui présente un risque moindre d'endommagement potentiel de la carotte. Ensuite, la carotte désaturée air/eau est plus facile à installer dans une cellule d'injection pour des expériences ultérieures. L'air peut être remplacé par de l'huile en mettant en oeuvre une séquence de déplacement miscible de C1, de cyclohexane et d'huile brute. En troisième lieu enfin, le calcul de la saturation est plus précis.

Selon une procédure standard de mise en oeuvre, trois mesures sont effectuées pour estimer la saturation moyenne finale : une mesure du volume expulsé hors de la carotte, une mesure du poids de la carotte (pour une estimation de la perte granulométrique) et une mesure RMN (avant et après centrifugation).

**Validation de la méthode**

Différentes expériences ont été conduites afin de montrer la validité de la méthode proposée. Dans un premier temps, nous avons vérifié l'effet de la plaque poreuse sur les profils de saturation. Ensuite, nous avons étudié la cinétique du processus de désaturation avec et sans plaque poreuse.

Profils de saturation par balayage CT

Des profils de saturation ont été mesurés sur deux carottes, l'une en grès et l'autre en carbonate, avec et sans plaque poreuse sous forme de support terminal (deux expériences réalisées de manière séquentielle, les carottes étant resaturées à 100 % entre-temps). L'effet de la plaque poreuse apparaît nettement à faible vitesse et à vitesse intermédiaire (figures 4A et 4B) pour le grès. A vitesse élevée, l'expérience menée sans plaque poreuse peut sembler donner un profil uniforme, mais ceci est dû à un manque de résolution de la mesure Scanner X. Avec la plaque poreuse, nous sommes sûrs d'obtenir un profil vraiment uniforme. Pour le carbonate compact également présenté sur les figures 4C et 4D, les profils obtenus à faible vitesse sont similaires à cause d'une durée de rotation trop courte. A la vitesse maximale, la différence est évidente. Toutefois, le profil obtenu en présence de plaque poreuse n'est pas entièrement uniforme. Dans ce cas, il convient de retourner la carotte et de poursuivre la centrifugation. Pour les deux carottes, la durée de rotation type était comprise entre 24 et 48 heures.

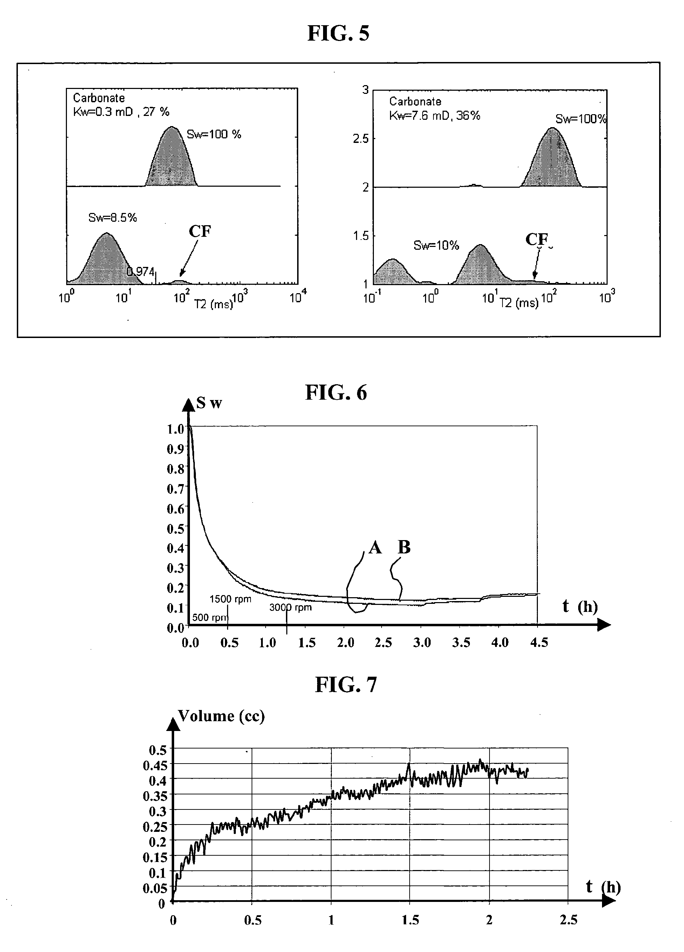

Une autre méthode permettant dé vérifier l'uniformité du profil de saturation consiste à effectuer des mesures de relaxation par RMN avant et après centrifugation (figure 5). Cette méthode de mesure rapide est également très précise pour estimer la saturation finale de systèmes air/eau. Lorsque la saturation est uniforme, la distribution de $T_2$ à (Swirr) ne devrait pas présenter de pic au temps de relaxation de la carotte saturée à 100 % parce que les temps de relaxation $T_2$ sont déplacés à des valeurs inférieures proportionnelles à la saturation (à partir de la relation fondamentale $T_2 \propto =V/S$ ; comme le montre la distribution, les principaux pics sont modifiés d'un facteur dix lorsque la saturation est descendue à 10 %). C'est ce que l'on observe pour le carbonate de faible perméabilité présenté sur la figure 5 (cadre de gauche). Pour la carotte présentant la plus forte perméabilité (cadre de droite), $T_2$ à saturation élevée ne s'étend pas dans le domaine de la carotte entièrement saturée, ce qui indique un pied capillaire négligeable.

Cinétique avec et sans plaque poreuse perforée

Nous avons testé la cinétique du processus de désaturation avec et sans plaque poreuse. Comme expliqué plus haut, les perforations ne devraient pas modifier sensiblement le débit. Ceci apparaît clairement lorsque l'on compare la saturation transitoire lors de la centrifugation de deux petites carottes de perméabilité moyenne (environ 180 mD, à noter que l'une de ces deux carottes est plus courte que l'autre de manière à avoir une perte de charge similaire). Une faible saturation est atteinte au bout de quelques heures dans les deux cas.

Dans l'exemple de la Fig.6 montrant une comparaison de la production transitoire avec et sans plaque poreuse pour deux petites carottes (air/eau), la vitesse de rotation était initialement fixée à 500 t/min (t=0), puis à 1500 t/min (t=0,5 h) et à 3000 t/min (t=1,25 h). Une faible saturation a été atteinte en quelques heures avec un déplacement dominé par la capillarité. Pour la plus grande carotte en grès, L=6 cm, Kw=194 mD, pour la plus petite, L=5 cm, Kw=171 mD.

Il est intéressant de connaître le temps typique nécessaire pour drainer le pied capillaire seul (figure 7). Au cours de cette expérience, une carotte de grès a d'abord été centrifugée au moyen de la technique PWC décrite dans :

-   Fleury et al., Proceedings of the International Symposium of the Society of Core Analysts, La Haye, September 14-16, 1998

et le niveau huile-eau a été maintenu en continu au voisinage de la face de sortie de la carotte (Rmax-d, figure 1). Au temps 0 (figure 7), le niveau est passé à Rmax et la production a été mesurée. Nous avons observé qu'une saturation stable est atteinte en quelques heures et que ce temps dépend essentiellement de la plaque poreuse, non de la carotte (cette observation s'applique ainsi également au drainage air/eau). La stabilisation de la saturation est assez rapide malgré la faible perméabilité de la plaque poreuse car seule une petite fraction de la carotte doit être drainée (approximativement une demi sphère d'un diamètre égal à la distance qui sépare les perforations).

En général, le temps de rotation dépend de différents paramètres (longueur, pression capillaire, perméabilité relative à l'eau à faible saturation) et il est difficile de faire une prédiction précise. L'expérience montre que, même pour une formation compacte, la saturation visée peut être atteinte en 48 heures.

Imbibition

On a décrit jusqu'ici une application de la méthode où l'on procédait au drainage de l'échantillon mis en contact du côté le plus éloigné (Rmax sur la Fig.1) de l'axe de rotation de la centrifugeuse avec une plaque poreuse mouillable par le premier fluide L1.

Il est possible de compléter l'étape précédente de drainage par une étape complémentaire d'imbibition. Cette fois, c'est le côté de l'échantillon préalablement drainé, le plus proche du centre de rotation de la centrifugeuse (Rmin Fig.1), qui est mis en contact avec une plaque poreuse mouillable par le deuxième fluide L2. Il peut s'agir par exemple d'une plaque en Téflon®. Elle est également percée de trous permettant le passage au travers du deuxième fluide L2.

Pour cette deuxième étape complémentaire, les mêmes précautions que pour la phase de drainage doivent être prises en ce qui concerne le nombre, le diamètre et la position des trous dans la plaque poreuse.

On peut observer, en guise de conclusion, que l'association centrifugeuse et plaque poreuse décrite exploite les avantages de ces deux techniques tout en éliminant leurs principaux inconvénients. Pour des carottes consolidées de perméabilité moyenne/élevée (> 10 mD), nous avons obtenu un profil uniforme, une faible saturation en eau étant atteinte en quelques heures/jours. Dans le cas d'une perméabilité faible à très faible, le profil de saturation peut être quasiment uniforme. Dans ce cas, il convient de procéder à une expérience supplémentaire qui consiste à retourner l'échantillon et à le centrifuger dans les mêmes conditions..

**Revendications**

1.   Méthode pour mesurer des caractéristiques physiques d'un échantillon solide poreux (S) saturé avec un premier fluide (L1) en réalisant des phases de drainage ou d'imbition, en présence d'un deuxième fluide (L2) de masse spécifique différente que celle du premier fluide, comportant l'utilisation d'un ensemble de centrifugation comportant au moins un godet (2) pour l'échantillon, adapté à appliquer à l'échantillon une force centrifuge orientée suivant la direction d'allongement et variable de manière à déplacer le premier fluide (L1) par le deuxième fluide (L2), et la détermination d'une valeur finale de saturation en fluide déplacé, **caractérisée en ce que** les phases de drainage ou d'imbition sont réalisées en appliquant une plaque poreuse contre une face terminale de l'échantillon traversée par le fluide déplacé, cette plaque poreuse étant mouillable par le fluide déplacé et perforée avec des perforations en nombre et avec une section adaptés pour permettre un drainage rapide du fluide contenu dans la carotte et obtenir un profil de saturation sensiblement uniforme.

2.   Méthode selon la revendication 1, dans laquelle la plaque poreuse perforée (1) est réalisée de manière à avoir une pression d'entrée supérieure à la pression capillaire la plus élevée imposée à la face de l'échantillon en contact avec la plaque poreuse.

3.   Méthode selon la revendication 1 ou 2, dans laquelle on réalise une phase de drainage d'un échantillon saturé par un premier liquide (L1) en le plaçant en contact avec une plaque poreuse perforée (1) mouillable par le premier fluide (L1) disposé du côté de l'échantillon le plus éloigné de l'axe de rotation de l'ensemble de centrifugation.

4.   Méthode selon la revendication 3, dans laquelle on utilise une plaque poreuse (1) réalisée dans un matériau tel que une céramique poreuse ou un ciment.

**5.** Méthode selon la revendication 3 ou 4, dans laquelle on réalise une phase d'imbibition de l'échantillon en le plaçant en contact avec une plaque poreuse perforée (1) mouillable par le deuxième fluide (L2) disposé du côté de l'échantillon le plus proche de l'axe de rotation de l'ensemble de centrifugation.

**6.** Méthode selon la revendication 5, dans laquelle on utilise une plaque poreuse perforée réalisée dans un matériau poreux et perméable mouillable-par le deuxième fluide tel que le Téflon.

**7.** Dispositif pour mesurer des caractéristiques physiques d'au moins un échantillon solide poreux (S) saturé avec un premier fluide (L1) en réalisant des phases de drainage ou d'imbibition, en présence d'un deuxième fluide (L2) de masse spécifique différente que celle du premier fluide, comportant un ensemble de centrifugation comprenant au moins un godet allongé (2) pourvu d'une chambre pour un échantillon (S), chaque godet étant fixé à l'extrémité d'un bras (20) solidaire d'un axe de rotation (19), des moyens moteurs (18) pour entraîner le bras en rotation et créer une force centrifuge orientée suivant la direction d'allongement du godet, et des moyens pour déterminer la valeur finale de la saturation en fluide déplacé, **caractérisé en ce qu'**il comporte une plaque poreuse (1) au contact de l'échantillon, cette plaque étant perforée avec des perforations en nombre et avec une section adaptés pour permettre un drainage rapide du fluide contenu dans la carotte et obtenir un profil de saturation sensiblement uniforme.

**8.** Dispositif selon la revendication 7, dans lequel la plaque poreuse est réalisée de manière à avoir une pression d'entrée supérieure à la pression capillaire la plus élevée avec la face de l'échantillon en contact avec la plaque poreuse.

## FIG. 1

## FIG. 2A

## FIG. 2B

## FIG. 2C

## FIG. 2D

# FIG. 3

# FIG. 4B

# FIG. 4A

# FIG. 4D

# FIG. 4C

## FIG. 5

## FIG. 6

## FIG. 7

FIG. 8